# EUROPEAN PATENT SPECIFICATION

(11) **EP 1 661 521 B1**
(45) Date of publication and mention of the grant of the patent: **30.04.2008**
(21) Application number: 04425883.8
(22) Date of filing: 25.11.2004
(51) Int. Cl.: A61B 17/34, A61B 10/00

(54) **Gripping member for biomedical needle**
Greifglied für biomezidinische Nadel
Elément de prise pour aiguille biomédicale

(43) Date of publication of application: 31.05.2006
(73) Proprietor: Carro, Luigi, 25030 Rudiano (Brescia) (IT)
(72) Inventor: Carro, Luigi, 25030 Rudiano (Brescia) (IT)
(74) Representative: Crippa, Paolo Ernesto

(56) References cited:
- US-A- 4 445 893
- US-A- 5 466 225
- US-A- 5 571 091
- US-A- 5 827 319

## Description

The present invention relates to a gripping member for a biomedical needle suitable for being used for various purposes, for example as a needle for aspirations, for anaesthesia, as an introducer needle, a guide needle, a needle for catheters and other uses.

As is known, gripping members for biomedical needles comprise a grip that can be connected to a hollow cannula and a cap that can be connected to a stylet suitable for being inserted into the cannula.

The cannula and stylet extend along a prevalent direction that is parallel to the direction of insertion of the needle itself. The stylet is suitable for closing the cavity of the cannula, at least partially, during its insertion.

The entrance ends of the cannula and stylet are suitably flared to facilitate penetration of the needle. Moreover, said ends of the stylet and cannula can have different geometries that are often asymmetric in relation to the prevalent direction. These geometries depend on the specific uses of the needle.

The gripping members for biomedical needles of the prior art do not guarantee relative safe positioning between the ends of the stylet and the cannula; consequently they are awkward for the surgeon to use and often particularly invasive in the insertion phase causing lesions to the patient. Such known gripping members are known from US 5466225 (closest prior art) and US 5571091.

The problem of the present invention is to realize a gripping member for a biomedical needle that resolves the problems mentioned with reference to the prior art.

These drawbacks and limitations are resolved with a gripping member for a biomedical needle in keeping with claim 1.

Other embodiments of the gripping member for a biomedical needle according to the invention are described in the following claims.

Further features and advantages of the present invention will be better understood from the description below of its preferred embodiments, which are not limiting, wherein:

Figure 1 represents a front view of a biomedical needle comprising a gripping member according to the present invention;

Figures 1A and 1C represent enlargements of detail A in figure 1, according to different embodiments;

Figures 1B and 1D represent enlargements of detail B in figure 1, according to different embodiments;

Figure 2 represents a front view of some elements of the biomedical needle in figure 1;

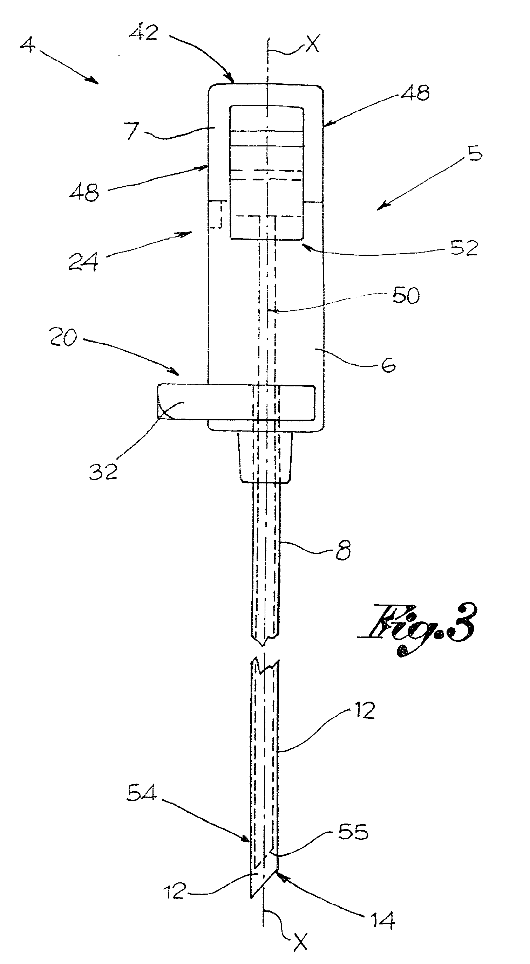

Figure 3 represents a side view of the needle in figure 1 from the side of the arrow III in figure 1;

Figure 4 represents a side view of the needle in figure 1, from the side of the arrow IV in figure 1, according to a further embodiment of the present invention;

Figures 5-7 represent front views of details of the needle in figure 1 in different phases of assembly;

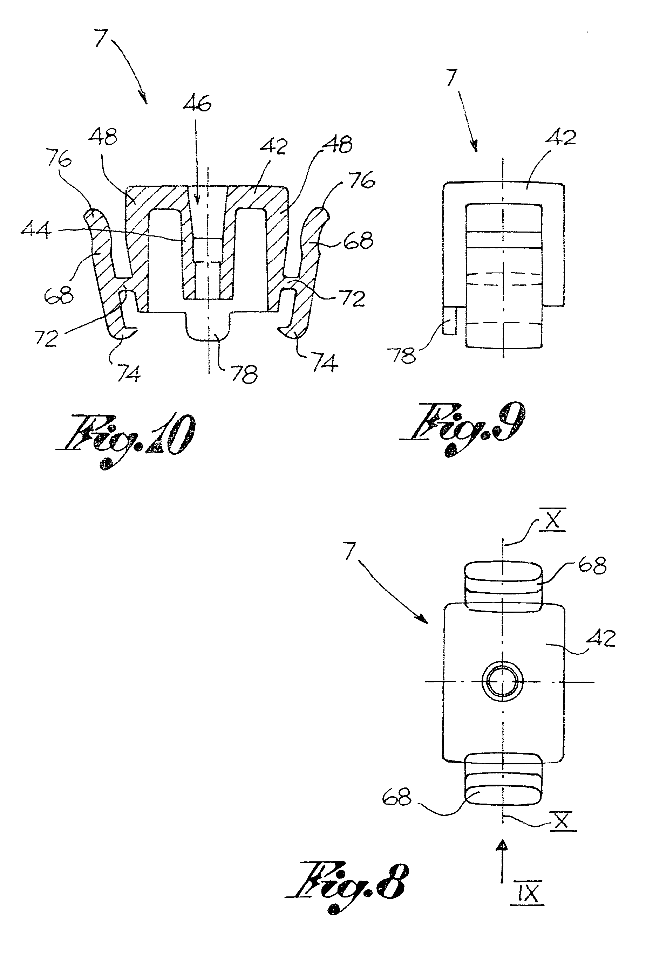

Figure 8 represents a top view from above of a detail of the needle in figure 1;

Figure 9 represents a side view of the detail in figure 8, from the side of the arrow IX in figure 8;

Figure 10 represents a section view of the detail in figure 8, along the X section plane in figure 8.

The elements or parts of elements in common between the embodiments described below will be indicated with the same numerical references.

With reference to the aforesaid figures, a biomedical needle has been generally indicated with reference numeral 4.

The biomedical needle 4 comprises a gripping member 5 suitable for allowing the needle to be grasped, and the relative insertion.

The gripping member 5 comprises a grip 6 to grasp the needle and a cap 7 that can be connected to said grip 6.

The biomedical needle 4 comprises a cylindrical cannula 8 that extends along a prevalent direction X, from a hooking end 9 to an insertion end 10. The cannula 8 contains a cylindrical cavity 12, preferably co-axial with said prevalent direction X. The cavity 12 extends from the hooking end 9 to the insertion end 10 that is consequently open.

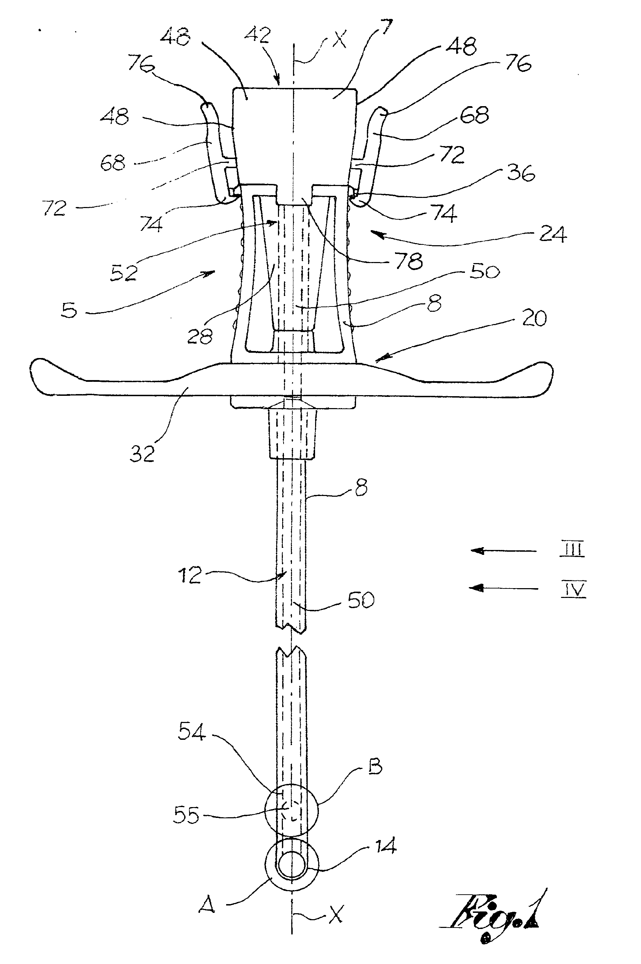

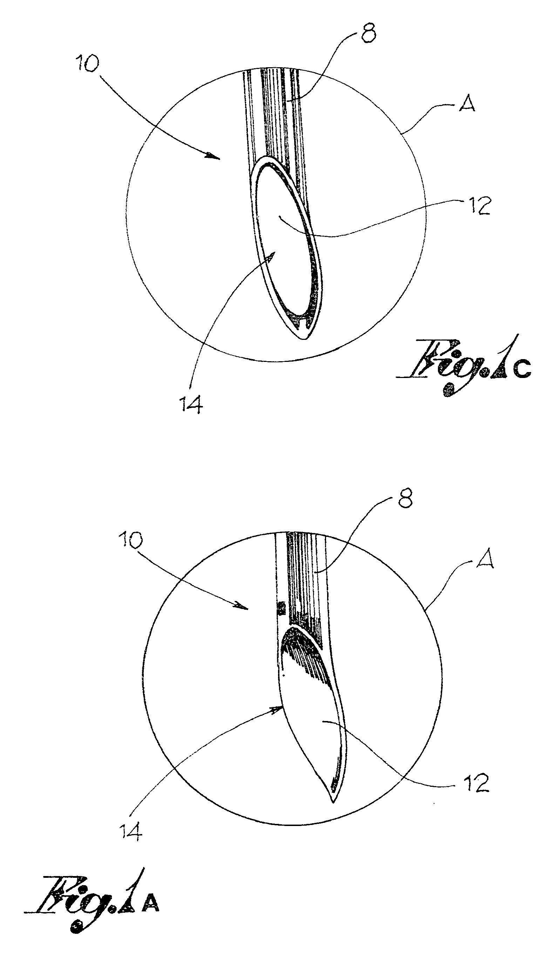

Depending on the specific use of the needle, the insertion end 10, can present different types of geometries, shown, for example in figure 1A and 1C.

The insertion end 10 preferably presents a first flaring 14 suitable for aiding the insertion and penetration of the cannula 8.

The hooking end 9 of the cannula 8 is integrally connected to a grip 6, level with a first end 20 of the grip itself. The grip 6 extends along said prevalent direction X from the first end 20 towards a second end 24.

The grip 6 presents an opening 28, which is advantageously conical, extending between said ends, in particular reducing in size from the first end 20 towards the second end 24, which is preferably coaxially in relation to the prevalent direction X.

On the part of the first end 20, the grip 6 comprises a plate 32, enbloc or which can be connected to it, for example by means of mortising that is suitable for aiding gripping of the needle with the fingers. The plate 32 is preferably perpendicular to the prevalent direction X.

On the part of the second end 24, the grip 6 advantageously comprises a collar 36, for example that is perimetrical in relation to said second end 24. The collar 36 is basically perpendicular to the prevalent direction X and has a transversal size, which is greater than the first end 20 of the grip 6. By transversal size we mean the overall size in relation to a surface perpendicular to the prevalent direction X.

According to one embodiment, the grip comprises an entrance 37 that is cylindrical for example, which projects from the second end 24, coaxially to the prevalent direction X.

On the whole the grip 6 is preferably prismatic with a rectangular base, said base being measured in relation to a surface perpendicular to said prevalent direction X.

Advantageously, a cap 7 can be connected to the grip that is suitable for closing the grip on the side of the second end 24.

According to one embodiment, the cap 7 comprises a cover 42 directly facing the entrance 37 that is basically level and perpendicular to the prevalent direction X. From the cover 42 a connecting portion 44, which is preferably symmetrical and coaxial in relation to the prevalent direction X and suitable for being inserted, at least partially, into the entrance 37 projects from the part of the connectable grip 6. The connecting portion 44 presents a hole 46 that is coaxial with the prevalent direction X.

According to one embodiment, the cap comprises a plurality of side walls 48 that project from the cover 42 to contain said connecting portion 44.

Advantageously, the cap 7 can be connected to a stylet 50 that is suitable for closing the cavity 12, at least partially.

The stylet 50 is basically a cylindrical body with an extended shape and coaxial with the cannula and the prevalent direction X.

The stylet 50 comprises an attachment portion 52 and a tip 54. The attachment portion 52 is suitable for being constrained to the cap 7 to make the stylet 50 integral with the cap 7. The attachment portion 52 is preferably inserted by means of mortising into the hole 46 of the connecting portion 44 of the cap.

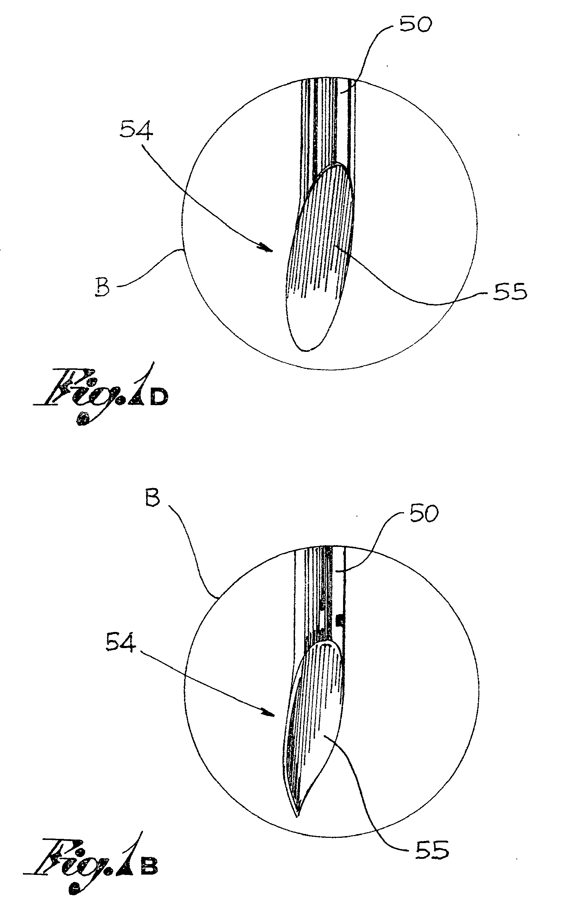

The tip 54 can have different geometries depending on the use of the needle. For example, as shown in figure 1B and 1D, the tip 54 can present a second basically level flaring 55, preferably with the same angle of inclination as the first flaring 14 of the insertion end 10 of the cannula 8.

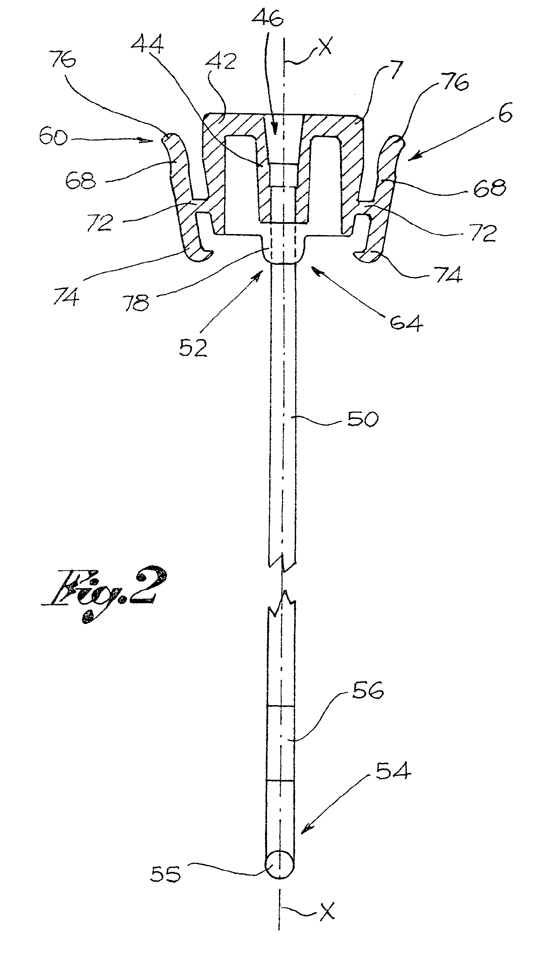

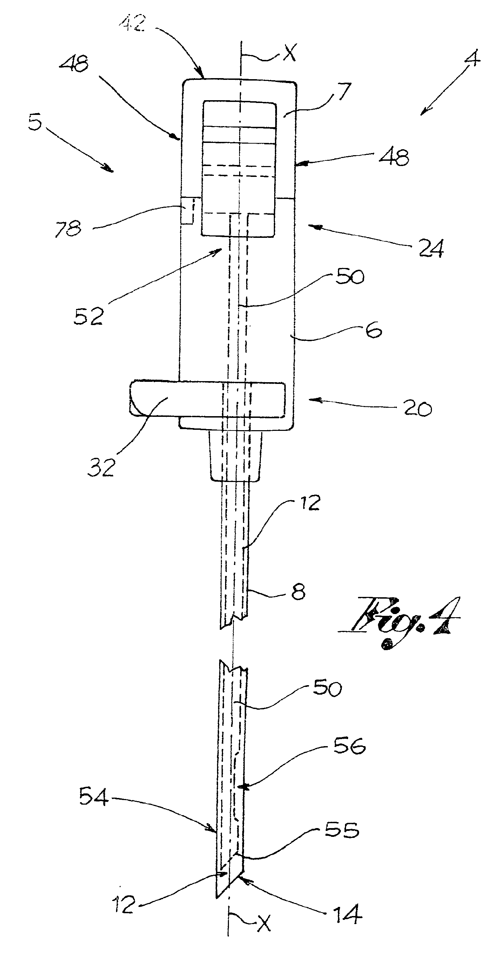

According to a further embodiment, shown for example in figures 2 and 4, near said tip 54 the stylet 50 comprises a levelling or slot 56 that is suitable, for example for taking a portion of tissue after inserting the needle and subsequently extracting the stylet 50. The levelling 56 is preferably aligned with the second flaring 55 of the tip 54, in relation to a centre plane of the stylet passing through the prevalent direction X.

Advantageously, the gripping member 5 comprises first and second constraining means 60, 64 between the cap 7 and the grip 6. Said first and second constraining means 60,64 can be both structurally separated and structurally integrated.

In particular, the first constraining means 60 are suitable for constraining the cap 7 to the grip 6 to block joint movements between the cap 7 and the grip 6, and consequently between the cannula and the stylet, in relation to the prevalent direction X.

According to one embodiment, the first constraining means 60 comprise at least one flap 68 constrained on a side wall 48 of the cap 7 by a separator 72 that basically realizes an elastic hinge, that is a fulcrum around which the flap 68 can rotate.

Advantageously, the flap 68 comprises a hooking portion 74 that is suitable for realising a shape coupling with the collar 36 of the grip 6. Said shape coupling preferably realizes a snap-on coupling.

Advantageously, the flap 68 extends beyond the separator 72 from the opposite side to the hooking portion 74, realizing a release portion 76.

According to a preferred embodiment, the cap 7 comprises a pair of flaps 68 arranged on side walls 48 of the cap 7 that are opposite in relation to the prevalent direction X. The collar 36 presents transversal dimensions, that is in relation to a surface perpendicular to the prevalent direction X, which are greater than the distance between the hooking portions 74 of the two opposite flaps 68 in a rest position.

The second constraining means 64 are advantageously suitable for constraining the relative position between the cap 7 and the grip 6 in a rotating manner in relation to the prevalent direction X; in other words they are suitable for blocking the relative rotation between the cap 7 and the grip 6, and consequently between the cannula 8 and the stylet 50, in relation to the prevalent direction X.

According to one embodiment, the second constraining means 64 comprise a tooth 78 that is integral with a side wall 48 of the cap 7 so it is directly facing the second end 24 of the grip 6 in relation to the prevalent direction X.

Advantageously, the grip 6, level with the second end 24, comprises a recess 80 suitable for containing said tooth 78 of the cap 7.

Inserting the tooth 78 into the relative recess 80 prevents the relative rotation between the cap 7 and the grip 6. Advantageously, the insertion of the tooth 78 is essentially coordinated with the hooking of the flaps 68 onto the relative collar 36.

Advantageously the second constraining means 64 realize a reference for the correct angular positioning between the tip 54 of the stylet 50 and the first end 20 of the cannula 8. In other words, the second constraining means 64 guarantee that after being hooked to the grip 6, the tip 54 of the stylet 50 and the first end 20 of the cannula 8 are suitably rotated, and constrained in this angular position, so that they cannot correspondingly rotate until the same constraining means 60, 64 are operated.

In other words again, the tooth 78 of the cap 7 is aligned with the second flaring 55 and the levelling 56 along a centre plane of the stylet 50 passing through the prevalent direction X, so that after hooking the cap 7 onto the grip 6, the insertion end 10 of the cannula 8 is automatically aligned with the second flaring 55 of stylet 50 and with the levelling 56, if any.

Furthermore, after the hooking, the first and second flaring 14, 55 are advantageously aligned.

Now a description will follow of the surgical technique of inserting and operating the biomedical needle 4 comprising a gripping member 5 according to the invention.

In an initial phase, the stylet 50 is inserted from the side of the tip 54 inside the grip 6, making the tip 54 of the stylet 50 pass through the connecting portion 44 of the grip 6. The conicity of the connecting portion 44 facilitates this operation.

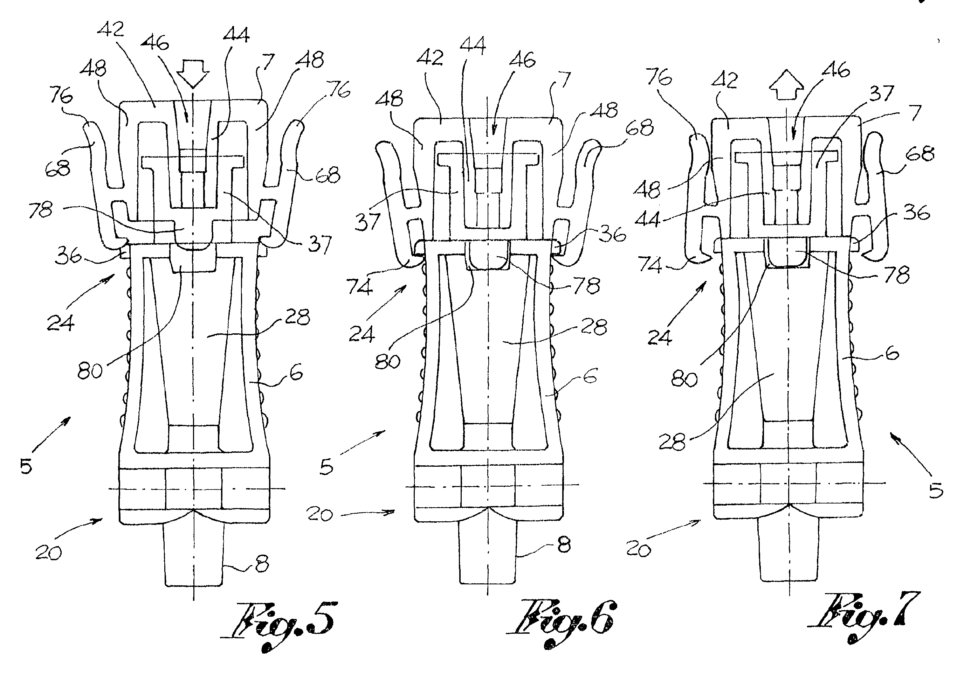

Then the cap 7 is brought into contact with the second end 24 of the grip 6, aligning the tooth 78 of the cap 7in relation to the recess 80 of the grip 6, as shown for example in figure 5.

The flaps 68 are pressed, in particular on the release portions 76 to facilitate coupling with the collar 36 of the grip 6 and at the same time the tooth 78 is pushed inside the recess 80, as shown for example in figure 6. The flaps 68 in fact rotate around the separator 72 to surpass the collar 36 and subsequently return to a rest position jointly blocking the hooking portions 74 and the collar 36.

Following the snap-on insertion, the cap 7 with the relative stylet 50 and the grip 6 with the relative cannula 8 are constrained both axially and angularly in relation to the prevalent direction X.

The insertion of the tooth 78 into the relative recess 80 guarantees the correct alignment between the tip 54 of the stylet 50 and the insertion end 10 of the cannula 8, so that the insertion of the needle 4 in a patient as little invasive as possible.

The operation of inserting the needle 4 is particularly convenient for the surgeon, because the surgeon does not need to worry about directing the cannula and stylet angularly and keeping them constrained, because they have both been blocked beforehand.

After inserting the needle 4 to the required depth, it is then possible to proceed with removing the stylet 50. This operation can be carried out by using the flaps 68 again to release them from the collar 36 of the grip 6, as shown for example in figure 7. This operation is particularly simple and can be carried out quickly using just one hand, for example by bringing the release portions 7 6 together jointly and consequently opening the hooking portions 74.

After removing the stylet 50, it is possible to connect any type of equipment to the grip 6, such as a device for example for sucking material or liquids or a device for injecting substances, a probe and the like.

As will be appreciated from this description, the gripping member described resolves the problems presented by the gripping members of the prior art.

In particular, the constraining means guarantee relative blocking between the cap and the grip and consequently a constraint between the relative positions of the cannula and the stylet.

In fact particularly during the insertion of the needle the ends of the stylet and the cannula must be able to be constrained in relation to each other, both in relation to an axial direction, parallel to the direction of insertion, to guarantee the exact, correct depth of insertion of the stylet inside the cannula, and in relation to an angular direction, that is the respective geometries of the ends of the stylet and cannula must be suitably rotated in relation to the prevalent direction of extension. The gripping member according to the present invention guarantees the correct relative positioning between said ends, so that the biomedical needle is as little invasive as possible.

Moreover, the rapid hooking system enables the user to hook and release the stylet rapidly without the risk of making movements with the needle that might cause lesions to the patient.

A further advantage lies in the fact that the rotational constraining means guarantee correct and safe alignment of the ends of the stylet and cannula without the possibility of errors on the part of the user.

Obviously an expert skilled in the art can make different modifications and variations to the above described gripping members to satisfy specific contingent needs.

For example, it is possible to connect the constraining means directly to the grip, realising both the collar and the relative slot for the tooth on the cap. Or it is possible to connect the flaps to the grip, keeping the tooth on the cap or vice versa.

The first constraining means can be realized by any snap-on hook, both in relation to a rounded collar and a prismatic collar.

The second constraining means can be made by any shape coupling between a protrusion and a slot connected to the cap and grip respectively.

Moreover, in figures 1 and 2 the constraining means are structurally different, because they are realized with different elements. They can also be integrated, for example, by including a tooth connected to at least one of the flaps, which is suitable for being inserted transversally into a slot open at the side of the grip or cap.

The number, shape and sizes of the flaps can vary from what is described and shown. The number, shape and sizes of the tooth and relative slot can vary in relation to what is described or shown. If a number greater than or equal to two are planned, the teeth are preferably arranged to define a single shared coupling position between the cannula and the stylet in order to direct them angularly.

Moreover, these and other embodiments are all contained in the scope of the invention, as defined by the following claims.

## Claims

1. Gripping member (5) for biomedical needle (4), comprising a grip (6) that is connectable to a cannula (8) with a cavity (12), and a cap (7) that can be connected to said grip (6), which is connectable to a stylet (50) suitable for being inserted into the cavity (12) of the cannula (8) to close said cavity (12), at least partially, the cannula (8) and the stylet (50) extending basically along a prevalent direction (X), said gripping member (5) comprising first and second constraining means (60,64) between the cap (7) and the grip (6) that are suitable for constraining the position of the cap (7) in relation to the grip (6) to block the joint positions between the cannula (8) and the stylet (50) both in parallel and turnably in relation to said prevalent direction (X), said grip (6) extending along said prevalent direction (X) from a first end (20), in correspondence of which the grip is connected to the cannula (8), to a second end (24) opposite to the first end, wherein said first constraining means (60) comprises at least one flap (68) connected elastically to said cap (7), said at least one flap comprising a hooking portion (74) that is suitable for realising a shape coupling with said grip (6), said second constraining means (64) comprises at least one tooth (78) and a recess (80), the recess (80) being suitable for containing said tooth (78), **characterised in that** said recess (80) is provided level with said second end (24), on the part of said second end (24), the grip (6), level with said recess (80), comprises a collar (36) which is basically perpendicular to the prevalent direction (X) and has a transversal size, in relation to a surface perpendicular to the prevalent direction (X), which is greater than the second end (24) of the grip (6), the collar (36) having transversal dimensions which are greater than the distance between said hooking portions (74) of two opposite flaps (68) in a rest position, the hooking portions realising a snap-on coupling with said collar (36) of the grip (6) in order to jointly blocking the hooking portions (74) and the collar (26), the hooking of the flaps onto the relative collar (36) being coordinated with the insertion of the tooth into the relative recess (80) on level with said second end.

2. Gripping member (5) according to claim 1, wherein said first and second constraining means (60, 64) are structurally separated.

3. Gripping member (5) according to claim 1, wherein said first and second constraining means (60, 64) are structurally integrated.

4. Gripping member (5) according to any one of claims 1 to 3, wherein said at least one flap (68) is keyed on a separator (72) of said cap (7) to realize an elastic hinge.

5. Gripping member (5) according to claim 4, I wherein said at least one flap (68) comprises a release portion (76), opposite with respect to said hooking portion (74).

6. Gripping member (5) according to any one of the claims from 1 to 5, wherein said cap (7) comprises two flaps (68) displaced at opposite ends of said cap (7), along the prevalent direction (X).

7. Gripping member (5) according to any one of the previous claims, wherein said second constraining means (64) comprise at least one tooth (78) suitable for realising a shape coupling with a corresponding recess (80) on said grip (6).

8. Gripping member (5) according to claim 7, wherein said at least one tooth (78) is aligned with a first flaring (14) of the cannula (8) and with a second flaring (55) of the stylet (50), so as to realise an angular reference between the cannula (89) and the stylet (50).

## Patentansprüche

1. Greifglied (5) für eine biomedizinische Nadel (4), umfassend einen Griff (6), der mit einer Kanüle (8) mit einem Hohlraum (12) verbindbar ist, sowie eine Kappe (7), die mit dem genannten Griff (6) verbunden werden kann, welches mit einem Stilett (50) verbindbar ist, das zum Einführen in den Hohlraum (12) der Kanüle (8) geeignet ist, um den genannten Hohlraum (12) zumindest teilweise zu verschließen, wobei sich die Kanüle (8) und das Stilett (50) im Wesentlichen entlang einer vorherrschenden Richtung (X) erstrecken, wobei das genannte Greifglied (5) erste und zweite Arretiermittel (60, 64) zwischen der Kappe (7) und dem Griff (6) umfasst, die geeignet sind, die Lage der Kappe (7) in Bezug auf den Griff (6) zu arretieren, um die gemeinsamen Positionen zwischen der Kanüle (8) und dem Stilett (50) sowohl parallel als auch drehbar in Bezug auf die genannte vorherrschende Richtung (X) zu verriegeln,
wobei sich der genannte Griff (6) entlang der genannten vorherrschenden Richtung (X) von einem ersten Ende (20), bei dem der Griff an der Kanüle (8) befestigt ist, zu einem zweiten Ende (24) erstreckt, das dem ersten Ende entgegengesetzt ist,
wobei die genannten ersten Arretiermittel (60) zumindest eine Klappe (68) umfassen, die elastisch mit der genannten Kappe (7) verbunden ist, wobei die genannte zumindest eine Klappe einen Hakenabschnitt (74) umfasst, der geeignet ist, eine formschlüssige Verbindung mit dem genannten Griff (6) zu realisieren, die genannten zweiten Arretiermittel (64) zumindest einen Zahn (78) und eine Ausnehmung (80) umfassen und die Ausnehmung (80) geeignet ist, den genannten Zahn (78) aufzunehmen,
**dadurch gekennzeichnet, dass**
die genannte Ausnehmung (80) auf gleicher Höhe mit dem genannten zweiten Ende (24) vorgesehen ist,
seitens des genannten zweiten Endes (24) der Griff (6), der auf gleicher Höhe mit der genannten Ausnehmung (80) ist, einen Bund (36) umfasst, der im Wesentlichen senkrecht zu der vorherrschenden Richtung (X) ist und in Bezug auf eine Fläche, die senkrecht zur vorherrschenden Richtung (X) ist, eine Größe in Querrichtung aufweist, die größer ist als das zweite Ende (24) des Griffes (6),
wobei der Bund (36) Querabmessungen aufweist, die größer sind als der Abstand zwischen den genannten Hakenabschnitten (74) der beiden entgegen gesetzten Klappen (68) in einer Ruheposition, wobei die Haken abschnitte eine Schnappverbindung mit dem genannten Bund (36) des Griffes (6) realisieren, um die Hakenabschnitte (74) und den Bund (36) gemeinsam zu verriegeln, wobei das Einhaken der Hebel an dem jeweiligen Bund (36) mit dem Einführen des Zahns in die betreffende Ausnehmung (80) auf der Höhe des genannten zweiten Endes koordiniert wird.

2. Greifglied (5) nach Anspruch 1,
wobei die genannten ersten und zweiten Arretiermittel (60, 64) konstruktiv voneinander getrennt sind.

3. Greifglied (5) nach Anspruch 1,
die genannten ersten und zweiten Arretiermittel (60, 64) einstückig aufgebaut sind.

4. Greifglied (5) nach einem der Ansprüche 1 bis 3,
wobei die genannte zumindest eine Klappe (68) an einem Separator (72) der genannten Kappe (7) befestigt ist, um ein biegsames Scharnier zu realisieren.

5. Greifglied (5) nach Anspruch 4,
wobei die genannte zumindest eine Klappe (68) einen Freigabeabschnitt (76) umfasst, der entgegengesetzt zu den Hakenabschnitten (74) vorgesehen ist.

6. Greifglied (5) nach einem der Ansprüche 1 bis 5,
wobei die genannte Kappe (7) zwei Klappen (68) umfasst, die an entgegen gesetzten Seiten der genannten Kappe (7) entlang der vorherrschenden Richtung (X) vorgesehen sind.

7. Greifglied (5) nach einem der vorhergehenden Ansprüche,
wobei die genannten zweiten Arretiermittel (64) zumindest einen Zahn (78) umfassen, der geeignet ist, eine formschlüssige Verbindung mit der korrespondierenden Ausnehmung (80) an dem genannten Griff (6) zu realisieren.

8. Greifglied (5) nach Anspruch 7,
wobei der genannte zumindest eine Zahn (78) zu einer ersten Öffnungserweiterung (14) der Kanüle (8) und zu einer zweiten Öffnungserweiterung (55) des Stiletts (50) ausgerichtet ist, um einen Winkelbezug zwischen der Kanüle (8) und dem Stilett (50) zu realisieren

## Revendications

1. Elément de prise (5) pour aiguille biomédicale (4), comprenant une prise (6) qui est connectable à une canule (8) ayant une cavité (12), et un capuchon (7) qui peut être connecté à la dite prise (6) et qui est connectable à un stylet (50) prévu pour être inséré dans la cavité (12) de la canule (8) de manière à fermer la dite cavité (12), au moins en partie, la canule (8) et le stylet (50) s'étendant essentiellement le long d'une direction principale (X), le dit élément de prise (5) comprenant un premier et un deuxième moyens de retenue (60, 64) entre le capuchon (7) et la prise (6) qui conviennent pour maintenir la position du capuchon (7) par rapport à la prise (6) afin de bloquer les positions de jonction entre la canule (8) et le stylet (50) à la fois en parallèle et angulairement par rapport à la dite direction principale (X),
ladite prise (6) s'étendant le long de la dite direction principale (X) à partir d'une première extrémité (20), en correspondance de laquelle la prise est connectée à la canule (8), jusqu'à une deuxième extrémité (24) opposée à la première extrémité,
dans lequel le premier moyen de retenue (60) comprend au moins un rabat (68) élastiquement relié au dit capuchon (7), le dit au moins un rabat comprenant une partie d'accrochage (74) qui convient pour effectuer un couplage de forme avec la dite prise (6), le dit deuxième moyen de retenue (64) comprend au moins une dent (78) et un évidement (80), l'évidement (80 étant prévu pour recevoir la dite dent (78),
**caractérisé en ce que**
le dit évidement (80) est prévu en correspondance de la dite deuxième extrémité (24),
du côté de la dite deuxième extrémité (24), la prise (6) au niveau du dit évidement (80) comprend une collerette (36) qui est sensiblement perpendiculaire à la direction principale (X) et qui a une dimension transversale, dans une surface perpendiculaire à la direction principale (X), qui est plus grande que la deuxième extrémité (24) de la prise (6),
la collerette (36) ayant des dimensions transversales qui sont plus grandes que la distance entre les dites parties d'accrochage (74) des deux rabats opposés (68) dans une position de repos, les parties d'accrochage produisant un accouplement à enclenchement avec la dite collerette (36) de la prise (6) afin d'assembler les parties d'accrochage (74) et la collerette (26), l'accrochage des rabats sur la collerette relative (36) étant coordonné avec l'insertion de la dent dans l'évidement relatif (80) au niveau de la dite deuxième extrémité.

2. Elément de prise (5) selon la revendication 1, dans lequel les dits premier et deuxième moyens de retenue (60, 64) sont structurellement séparés.

3. Elément de prise (5) selon la revendication 1, dans lequel les dits premier et deuxième moyens de retenue (60, 64) sont structurellement intégrés.

4. Elément de prise (5) selon une quelconque des revendications 1 à 3, dans lequel le dit au moins un rabat (68) est claveté sur un séparateur (72) du dit capuchon (7) pour réaliser une charnière élastique.

5. Elément de prise (5) selon la revendication 4, dans lequel le dit au moins un rabat (68) comprend une partie de libération (76) à l'opposé de la dite partie d'accrochage (74).

6. Elément de prise (5) selon une quelconque des revendications 1 à 5, dans lequel le dit capuchon (7) comprend deux rabats (68) décalés à des extrémités opposées du dit capuchon (7), le long de la direction principale (X).

7. Elément de prise (5) selon une quelconque des revendications précédentes, dans lequel le dit deuxième moyen de retenue (64) comprend au moins une dent (78) convenant pour effectuer un couplage de forme avec un évidement correspondant (80) sur la dite prise (6).

8. Elément de prise (5) selon la revendication 7, dans lequel la dite au moins une dent (78) est alignée avec un premier évasement (14) de la canule (8) et avec un deuxième évasement (55) du stylet (50), afin de réaliser une référence angulaire entre la canule (8) et le stylet (50).
